(19) Europäisches Patentamt
European Patent Office
Office européen des brevets

(11) **EP 2 959 883 A1**

(12) **EUROPEAN PATENT APPLICATION**
published in accordance with Art. 153(4) EPC

(43) Date of publication:
**30.12.2015 Bulletin 2015/53**

(21) Application number: **14754873.9**

(22) Date of filing: **18.02.2014**

(51) Int Cl.:
***A61K 8/18*** *(2006.01)*  ***A61K 31/164*** *(2006.01)*
***A61K 31/20*** *(2006.01)*  ***A61K 47/38*** *(2006.01)*
***A61Q 19/00*** *(2006.01)*

(86) International application number:
**PCT/KR2014/001289**

(87) International publication number:
**WO 2014/129779 (28.08.2014 Gazette 2014/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.02.2013 KR 20130017446**
**17.02.2014 KR 20140017627**

(71) Applicant: **Neopharm Co., Ltd.**
**Daejeon 305-510 (KR)**

(72) Inventors:
• **KIM, Su-Hwan**
**Daejeon 302-881 (KR)**

• **LEE, Sin-Hee**
**Daejeon 300-737 (KR)**
• **KIM, Yoon**
**Daejeon 306-778 (KR)**
• **JEONG, Se-Kyoo**
**Daejeon 302-734 (KR)**

(74) Representative: **Von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

(54) **COMPOSITION EXTERNALLY USED FOR SUBACID SKIN AND COSMETIC MATERIAL INCLUDING SAME**

(57) The present invention relates to a composition with a pH of 3-5, preferably 3-4.5 externally used for a subacid skin composed of a multi-lamellar emulsion including pH modulation agent and a polymeric thickening agent, and a composition externally used for a subacid skin according to the present invention maintains stably a multi-lamellar emulsion with a skin barrier function composed of a large amount of a lipophilic skin care material at a relatively low pH condition so as to enhance or recover the skin barrier function through normalizing the skin pH, thus recovering and maintaining a healthy skin, making the skin effectively moisturized, maintaining a proper viscosity so as to prevent the phase separation, and having almost no skin penetration so as to eliminate the problem of stimulating the skin.

**Fig. 2**

**Description**

**Technical Field**

The present invention relates to a weakly acidic composition for skin external application, a multi-lamellar emulsion containing a ceramide, a sterol, a fatty acid, a higher alcohol and an emulsifier, a pH adjuster and a polymeric thickener, and a cosmetic formulation comprising the composition. More particularly, the present invention relates to a weakly acidic composition for skin external application, in which a multi-lamellar emulsion containing large amounts of dermatologically useful oleophilic substances and having skin barrier function is stably maintained at a relatively low pH, so that the composition can normalize the pH of the skin to enhance or restore the skin's barrier function to thereby restore and maintain the skin in a healthy state, and can effectively moisture the skin, and the composition is maintained at suitable viscosity so as to be effectively prevented from phase separation, and does not penetrate the skin, and thus causes no skin irritation, and to a cosmetic formulation comprising the composition.

**Background Art**

The epidermis of the skin consists of stratum corneum, stratum lucidum, stratum granulosum, stratum spinosum and stratum basale layers. Among these layers, the stratum corneum (horny layer) forms the outermost layer of the skin, and is one of the most important portions of the human body, which functions as a primary barrier to prevent the loss of water from the body and protect the body from external harmful contaminants.
The horny layer is composed of keratinocytes and intercellular lipids. Herein, keratinocytes are cells derived from the stratum basale (basal layer) of the epidermis, and contain the protein keratin. Intercellular lipids are present between these keratinocytes and composed of ceramides, cholesterols, fatty acids, etc. These are present as a multi-lamellar structure and play a very important role in skin moisturization and protection by enhancement of the skin barrier, and the structure thereof can be observed by polarized optical microscopy.
This multi-lamellar structure was described first by Peter M. Elias through the "Brick and Mortar" model, and then proven through studies (The Characterization of Molecular Organization of multilamellar Emulsions Containing Pseudoceramide and Type III Synthetic Ceramide. J Invest Dermatol. 2003 Oct; 121 (4) :794-801.).
Meanwhile, it was reported that, when the pH of the skin is unsuitable or changes to neutral or alkaline pH values due to frequent washing or disease, the activity of lipase will decrease, and thus the synthesis of ceramides, cholesterols and fatty acids, which form intercellular lipids, will decrease, causing damage to the skin barrier (Effects of soaps and detergents on skin surface pH, stratum corneum hydration and fat content in infants. Dermatology 1997; 195: 258-262). Moreover, it was reported that a neutral or weakly alkaline pH can result in the formation of conditions advantageous for the proliferation of skin flora, thus causing various skin diseases (The effect of detergents on skin pH and its consequences. Clin Dermatol. 1996; 14(1): 23-27.). In addition, it was reported that an increase in the pH of the skin can result in the weakening of the skin barrier, thus causing skin troubles, sensitive skin, dry skin, and even atopy (Abnormal skin barrier in the pathogenesis of atopic dermatitis. Peter M. Elias, M.D. University of California, San Francisco, CA 94121).
The skin of the human body should be maintained at a weakly acidic pH for the following reasons. First, at a weakly acidic pH, lipase that is involved in the synthesis of the constituent components of intercellular lipids forming the skin barrier is activated. This results in an increase in the synthesis of ceramides, cholesterols and fatty acids, which are the constituent components of intercellular lipids (Stratum Corneum pH in Atopic Dermatitis. Am J Clin Dermatol 2004; 5 (4): 217-223). Second, the proliferation of normal flora on the skin surface is suppressed, the invasion of external bacteria is prevented, and thus the normal function of the skin barrier is maintained. Accordingly, maintaining the pH of the skin at a weakly acidic pH is important to make the skin moist, elastic and soft (Skin barrier: Cutaneous barriers in defense against microbial invasion, Anna Di Nardo and Richard L. Gallo pp. 364-365).
Thus, a composition for skin external application, which has a multi-lamellar structure and a relatively weakly acidic pH, preferably a pH of about 3-5, more preferably about 3-4.5, has a function of enhancing the skin barrier, and also has the effect of maintaining the skin at a normal pH or restoring the skin to a normal pH. Thus, this composition makes a skin, which has damaged or reduced skin barrier function, healthy.
In the prior art, many cosmetic products for restoring the skin barrier function, which contain various ceramides, were disclosed, but most of these cosmetic products did not have a multi-lamellar structure, but contained active ingredients which were simply added and mixed, and thus the effects thereof were insignificant.
In addition, a multi-lamellar emulsion type cosmetic product containing ceramide was proposed by the applicant and is being sold, but the pH thereof is close to a neutral pH and is not weakly acidic.
However, if an organic acid is added to a cosmetic formulation to make the cosmetic formulation weakly acidic, the viscosity of the formulation will necessarily be reduced, and if the viscosity is reduced, the phase stability of the formulation will be reduced so that the time-dependent stability of the formulation will be reduced, thus causing phase separation. In addition, serious damage to the characteristic liquid crystal structure of the multi-lamellar emulsion will be caused. These problems are not satisfactorily solved even when a thickener is added.

If alpha-hydroxy acid (AHA) or beta-hydroxy acid (BHA) is added as the organic acid as described above, AHA in the former case can penetrate the skin to cause skin irritation and increase the photosensitivity of the skin. In addition, at a pH of less than, it is difficult to obtain a stable emulsion formulation. In the latter case, skin irritation is low, but still remains, and at a pH of less than 5, it is difficult to obtain a stable emulsion formulation.

If an acidic thickener is used in a formulation without adding the organic acid as described above, the pH of the formulation can become weakly acidic. However, at this weakly acidic pH, there are serious problems in that the inherent liquid crystal structure of the multi-lamellar structure is damaged and the viscosity of the formulation is also reduced. A reduction in the viscosity of the formulation leads to a decrease in phase stability, resulting in phase separation.

Accordingly, there has been a need for the development of an effective cosmetic formulation that can effectively restore or enhance the skin barrier function of a skin having damaged or reduced skin barrier function by normalizing the pH of the skin and, at the same time, has high phase stability and causes little or no skin irritation.

## Disclosure

### Technical Problem

It is a first object of the present invention to provide a weakly acidic composition for skin external application, in which a multi-lamellar emulsion containing large amounts of dermatologically useful oleophilic substances and having a skin barrier function is stably maintained at a relatively low pH, so that the composition can normalize the pH of the skin to enhance or restore the skin's barrier function to thereby restore and maintain the skin in a healthy state and can effectively moisturize the skin.

A second object of the present invention is to provide a weakly acidic composition for skin external application, which can be maintained at suitable viscosity, and thus has high phase stability and high time-dependent stability so as to be effectively prevented from phase separation.

A third object of the present invention is to provide a weakly acidic composition for skin external application, which has low skin penetrability, and thus causes little or no skin irritation.

A fourth object of the present invention is to provide a weakly acidic cosmetic formulation containing, as an active ingredient, the weakly acidic composition for skin external application described in the above objects.

### Technical Solution

In order to accomplish the above objects, in accordance with a preferred aspect of the present invention, there is provided a weakly acidic composition for skin external application, which comprises: a multi-lamellar emulsion containing a ceramide, a sterol, a fatty acid, a higher alcohol and an emulsifier; a pH adjuster; and a polymeric thickener, the composition having a pH of 3-5, preferably 3-4.5.

In accordance with a preferred embodiment of the present invention, there is provided a weakly acidic composition for skin external application wherein the multi-lamellar emulsion is contained in an amount of 1.0-50.0 wt% based on the total weight of the composition.

In accordance with another preferred embodiment of the present invention, there is provided a weakly acidic composition for skin external application wherein the multi-lamellar emulsion contains 0.01-5 wt% of the ceramide, 0.1-5 wt% of the sterol, 1-20 wt% of the fatty acid, 1-20 wt% of the higher alcohol, and 1-15 wt% of the emulsifier.

In accordance with still another preferred embodiment of the present invention, there is provided a weakly acidic composition for skin external application wherein the ceramide is at least one selected from the group consisting of ceramide-1, ceramide-3, ceramide-3B, ceramide-4, ceramide-6, myristoyl/palmitoyl oxostearamide/arachamide MEA, and dihydroxyisopropyl palmoylpalmamide; the sterol is at least one selected from the group consisting of cholesterol, cholesteryl acetate, cholesteryl sulfate, and phytosterol; the fatty acid or fatty alcohol is a fatty acid or fatty alcohol having 14 to 22 carbon atoms; and the emulsifier is at least one selected from the group consisting of hydrogenated lecithin, polyglyceryl fatty acid ester, sorbitan fatty acid ester, polyoxyethylene glyceryl fatty acid ester, alkyl glucoside, and polyoxyethylene sorbitan fatty acid ester.

In accordance with still another preferred embodiment of the present invention, there is provided a weakly acidic composition for skin external application wherein the pH adjuster is carbomer.

In accordance with still another preferred embodiment of the present invention, there is provided a weakly acidic composition for skin external application wherein the polymeric thickener is at least one selected from the group consisting of hydroxyethyl cellulose, hydroxypropyl cellulose, hydroxypropylmethyl cellulose stearoxy ether, ammonium acryloyldimethyl taurate/VP copolymer, xanthan gum, and acrylate/alkyl acrylate crosspolymer.

In another preferred aspect of the present invention, there is provided a cosmetic formulation containing 1-99 wt% of the weakly acidic composition for skin external application.

## Advantageous Effects

A weakly acidic composition for skin external application according to the present invention comprises a multi-lamellar emulsion containing large amounts of dermatologically useful oleophilic substances and having a skin barrier function, which is stably maintained even at a relatively low pH. Thus, the composition can normalize the pH of the skin to enhance or restore the skin's barrier function to thereby restore and maintain the skin in a healthy state and can effectively moisturize the skin. In addition, the composition according to the present invention can be maintained at suitable viscosity, and thus has high phase stability and high time-dependent stability so as to be effectively prevented from phase separation. Furthermore, the composition according to the present invention has low skin penetrability, and thus causes little or no skin irritation. Thus, a weakly acidic cosmetic formulation comprising the composition according to the present invention contributes to normalizing the pH of a skin having damaged or reduced skin barrier function, activates lipase that is involved in the synthesis of lipids functioning as a skin barrier, thereby making keratinocytes stable, and inhibits the abnormal proliferation or invasion of skin flora to thereby maintain the skin in a healthy state.

## Description of Drawings

FIGS. 1 to 4 are polarized microscope photographs of liquid crystals, which show the multi-lamellar emulsion structures of weakly acidic compositions for skin external application according to Examples of the present invention.
FIGS. 5 to 9 are polarized microscope photographs of weakly acidic compositions for skin external application according to Comparative Examples.

## Best Mode

The present invention provides a weakly acidic composition for skin external application, which comprises: a multi-lamellar emulsion containing a ceramide, a sterol, a fatty acid, a higher alcohol and an emulsifier; a pH adjuster; and a polymeric thickener, the composition having a pH of 3-5, preferably 3-4.5.

## Mode for Invention

Hereinafter, the present invention will be described in detail.

The multi-lamellar emulsion that is used in the weakly acidic composition for skin external application according to the present invention contains a ceramide, a sterol, a fatty acid, a higher alcohol and an emulsifier.

The ceramide that is used in the multi-lamellar emulsion may be any one or more selected from among natural ceramides, including ceramide-1, ceramide-3, ceramide-3B, ceramide-4 and ceramide-6, and pseudoceramides synthesized by the applicant, for example, myristoyl/palmitoyl oxostearamide/arachamide MEA (trade name: PC-9S) and dihydroxy isopropyl palmoyl palmamide (trade name: PC-5). The content of the ceramide in the multi-lamellar emulsion is preferably 0.01-5 wt%.

The sterol that is used in the present invention has a fused ring structure, and thus contributes to maintaining the multi-lamellar emulsion. The sterol may be one or more selected from among cholesterol and its derivatives, including cholesteryl acetate, cholesteryl sulfate, stigmasterol, beta-sitosterol, ergosterol, and plant-derived phytosterol that is a mixture thereof. The content of the sterol in the multilamellar emulsion may be 0.1-5 wt%.

The fatty alcohol that is used in the present invention may be any one or more selected from among fatty alcohols having 14 to 22 carbon atoms, and examples thereof include cetyl alcohol, stearyl alcohol, cetearyl alcohol, behenyl alcohol, etc. The content of the fatty alcohol in the multilamellar emulsion is preferably 1-20 wt%.

The fatty acid that is used in the present invention may be any one or more selected from among fatty acids having 14 to 22 carbon atoms, and examples thereof include myristic acid, palmitic acid, stearic acid, oleic acid, etc. The content of the fatty acid in the multi-lamellar emulsion is preferably 1-20 wt%.

The emulsifier that is used in the present invention may be selected from among hydrogenated lecithin, polyglyceryl fatty acid ester, sorbitan fatty acid ester, polyoxyethylene glyceryl fatty acid ester, alkyl glucoside, and polyoxyethylene sorbitan fatty acid ester. The content of the emulsifier in the multi-lamellar emulsion is preferably 1-15 wt%.

A multi-lamellar emulsion made of such components shows particular lamellar crystal phase when observed with a polarized microscope.

The role of the ceramide and the sterol in the above-described multi-lamellar emulsion is important.

This role will be explained below, and Table 1 below shows several examples of the multi-lamellar emulsion and the results of observation of formed liquid crystal phases.

The pH adjuster that is used in the present invention is particularly preferably carbomer having a carboxyl group in the structure, instead of an organic acid. Carbomer is a synthetic polymer made of acrylic acid or its derivative, and has an acrylic acid content of 56-58 wt%. An aqueous dispersion of 0.5-1.0 (w/v)% of carbomer has a pH of 2.5-3.5, and is effectively used to reduce the pH of the weakly acidic composition for skin external application according to the present invention. Meanwhile, caution is required because carbomer can cause the loss or reduction of consistency in the presence of a cationic polymer, acid or electrolyte.

If the carbomer is neutralized, it acts as a thickener, but the carbomer is not neutralized, it acts to stabilize the emulsion. In addition, because the carbomer is a polymer, it does not penetrate the skin, and thus does not cause side effects such as skin irritation.

If the carbomer is used as a pH adjuster or a pH lowering agent in the multi-lamellar emulsion, a reduction in the viscosity of the emulsion will occur to make it difficult to stabilize the emulsion. This problem cannot be solved with a conventional polymeric thickener.

The present inventors have conducted experiments on a wide range of selected polymeric thickeners, and as a result, have found that the use of hydroxyethyl cellulose, hydroxypropyl cellulose, hydroxypropylmethyl cellulose stearoxy ether, ammonium acryloyldimethyl taurate/VP copolymer, acrylate/alkyl acrylate crosspolymer and xanthan gum alone or in combination, preferably one or more selected from hydroxypropylmethyl cellulose stearoxy ether, ammonium acryloyldimethyl taurate/VP copolymer and xanthan gum, particularly hydroxypropylmethyl cellulose stearoxy ether and/or ammonium acryloyldimethyl taurate/VP copolymer, can provide satisfactory results.

The content of the carbomer in the emulsion may vary depending on a desired pH, but is preferably 0.05-2 wt%. If the content of the carbomer is lower than the lower limit of the above range, a sufficient pH-lowering effect cannot be obtained, and if the content of the carbomer is higher than the upper limit of the above range, the pH and viscosity of the emulsion will deviate from desired values.

The thickener can be selectively used so that it can adjust the emulsion to a desired viscosity.

The weakly acidic composition for skin external application according to the present invention can be provided as a cosmetic formulation such as a basic cosmetic product or a makeup cosmetic product. Formulations of the basic cosmetic product include lotion, cream, essence, skin lotion, pack, massage cream, cleansing foam, cleansing water, cleansing oil and the like.

In addition, it is to be understood that additives that are generally used in the cosmetic field can be suitably added according to the intended use.

Examples and Experimental Examples

Hereinafter, the present invention will be described in detail with reference to examples, comparative examples and experimental examples. It is to be understood, however, that these examples are for illustrative purposes and are not intended to limit the scope of the present invention.

1. Preparation of multi-lamellar emulsion pre-mix

In order to examine the effects of ceramide, sterol and fatty acid on the formation of a multi-lamellar emulsion, an emulsion was prepared, and the liquid crystal phase thereof was observed.

Whether the prepared emulsion easily formed a lamellar liquid crystal phase was evaluated based on the shape and frequency of a characteristic Maltese cross texture observed by a polarized microscope (Optiphto-2, manufactured by Nikon).

Criteria for the evaluation were as follows:

◎: the percentage of the liquid crystal phase in formed emulsion particles is more than 80%;
○: the percentage of the liquid crystal phase in formed emulsion particles is 50-80%;
Δ : the percentage of the liquid crystal phase in formed emulsion particles is less than 50%;
x: the liquid crystal phase is not substantially formed.

Oil phase components and an emulsifier were dissolved uniformly and emulsified at a temperature of 60 to 80°C for 10 minutes while hot purified water was added thereto. After completion of the emulsification, the emulsion was cooled to room temperature while it was stirred.

Prepared formulations and the results of observation of the liquid crystal phase are shown in Table 1 below.

Table 1: Formulation of multi-lamellar emulsion

| Components | Examples 1 to 4 | | | | Comparative Examples 1 to 4 | | | |
|---|---|---|---|---|---|---|---|---|
| | Example 1 | Example 2 | Example 3 | Example 4 | Comp. Example 1 | Comp. Example 2 | Comp. Example 3 | Comp. Example 4 |
| PC-9S | 3 | 3 | 3 | - | - | 0.1 | - | - |
| Ceramide 3 | - | - | - | 3 | - | - | 0.1 | - |
| Phytosterol | 2 | 2 | 2 | 2 | - | - | - | 0.1 |
| Stearic acid | 5 | 5 | 5 | 5 | 5 | 5 | 5 | 5 |
| Cetearyl alcohol | 10 | 10 | 10 | 10 | 10 | 10 | 10 | 10 |
| Capric/caprylic triglyceride | 10 | 10 | 10 | 10 | 10 | 10 | 10 | 10 |
| Glyceryl fatty acid ester | 5 | 5 | 5 | 5 | 5 | 5 | 5 | 5 |
| Polyglyceryl fatty acid ester | 5 | - | - | - | 5 | - | - | - |
| Sorbitan fatty acid ester | - | 5 | - | - | - | 5 | - | - |
| Hydrogenated lecithin | - | - | 5 | - | - | - | 5 | - |
| PEG glyceryl fatty acid ester | - | - | - | 5 | - | - | - | 5 |
| Purified water | To 100 | To 100 | To 100 | To 100 | To 100 | To 100 | To 100 | To 100 |
| Results of liquid crystal phase polarized microscope | ◎ | ◎ | ◎ | ○ | x | Δ | Δ | Δ |

## 2. Preparation of emulsion composition having low pH

Using the multi-lamellar emulsions prepared as shown in Table 1 above, weakly acidic compositions for skin external application were prepared, and the pH, viscosity, liquid crystal phase, time-dependent stability, skin irritability and moisturizing ability of the prepared compositions were evaluated.

According to the components and contents shown in Table 2 below, the components were placed in a mixer, and heated, stirred and cooled, thereby preparing cosmetic formulations of the Examples and the Comparative Examples.

Table 2: Formulations for evaluation

| Components | Example 5 to 8 | | | | Comparative Example 5 to 13 | | | | | | | | |
|---|---|---|---|---|---|---|---|---|---|---|---|---|---|
| | 5 | 6 | 7 | 8 | 5 | 6 | 7 | 8 | 9 | 10 | 11 | 12 | 13 |
| Composition of Example 1 | 30 | - | - | - | 30 | - | - | - | - | - | - | - | 30 |
| Composition of Example 2 | - | 30 | - | - | - | 30 | - | - | - | - | - | - | - |
| Composition of Example 3 | - | - | 30 | - | - | - | 30 | - | - | - | - | - | - |
| Composition of Example 4 | - | - | - | 30 | - | - | - | 30 | - | - | - | - | - |
| Composition of Comparative Example 1 | - | - | - | - | - | - | - | - | 30 | - | 30 | - | - |
| Composition of Comparative Example 2 | - | - | - | - | - | - | - | - | - | 30 | - | 30 | - |
| Glycerin | 10 | 10 | 10 | 10 | 10 | 10 | 10 | 10 | 10 | 10 | 10 | 10 | 10 |
| Carbomer | 0.4 | 0.4 | 0.4 | 0.4 | - | - | - | - | 0.4 | 0.4 | - | - | - |
| Lactic acid | - | - | - | - | 0.8 | 0.8 | 0.8 | 0.8 | 0.2 | 0.2 | 0.8 | 0.8 | - |
| Xanthan gum | 0.1 | - | - | - | 0.3 | - | 0.3 | - | 0.3 | 0.3 | 0.3 | | 0.3 |
| Hydroxypropyl methyl cellulose stearoxy ether | 0.1 | 0.3 | - | 0.1 | - | - | - | - | - | - | - | - | - |
| Ammonium acryloyl dimethyl tauate/VP copolymer | 0.1 | - | 0.3 | 0.2 | - | - | - | - | - | - | - | - | - |
| Preservative | q.s. | q.s. | q.s. | q.s. | q.s. | q.s. | q.s. | q.s. | q.s. | q.s. | q.s. | q.s. | q.s. |
| Sodium hydroxide | - | - | - | - | - | - | - | - | - | - | - | - | q.s. |
| Purified water | to 100 | to 100 | to 100 | to 100 | to 100 | to 100 | to 100 | to 100 | to 100 | to 100 | to 100 | to 100 | to 100 |

1) Evaluation of liquid crystal phase

According to the above evaluation criteria, the liquid crystal phase of the prepared samples was observed with a polarized microscope.

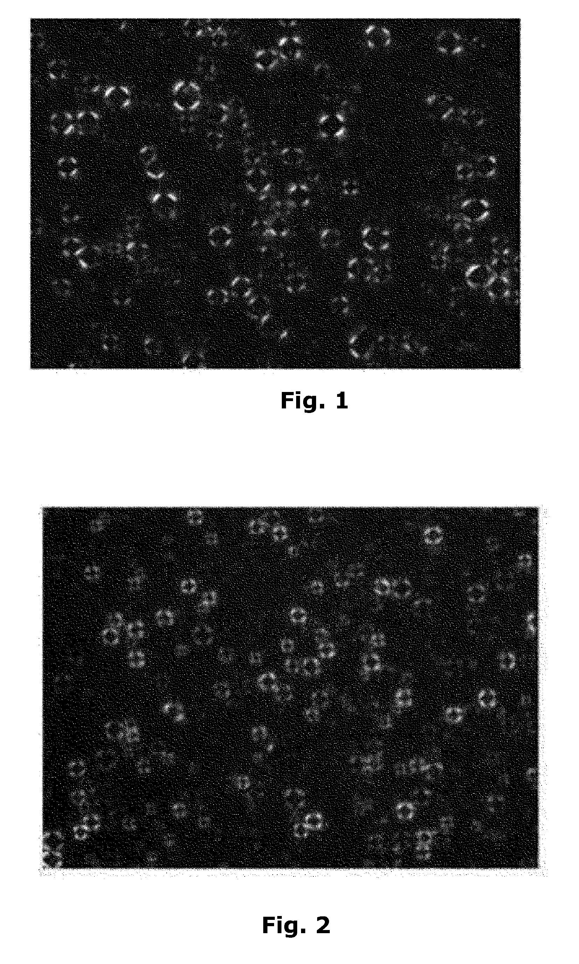

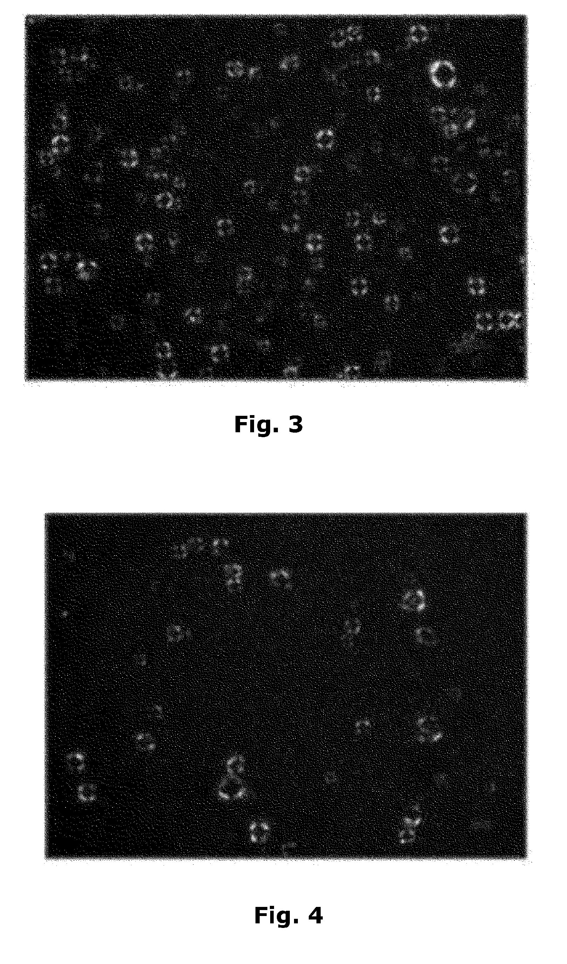

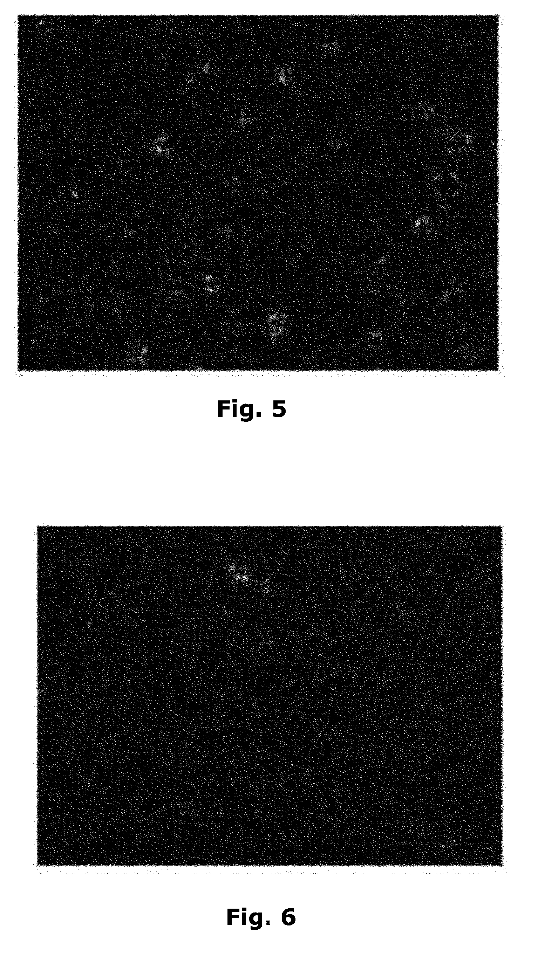

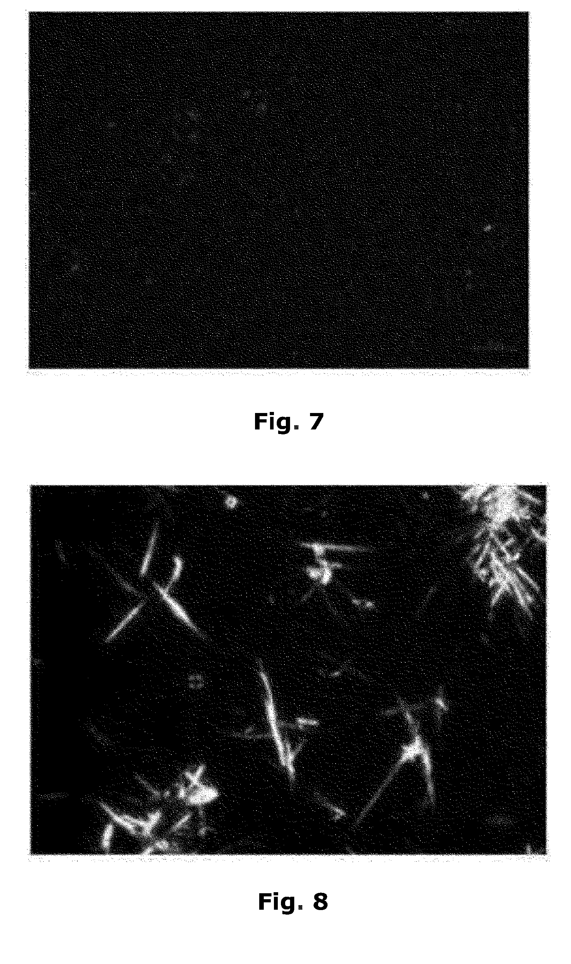

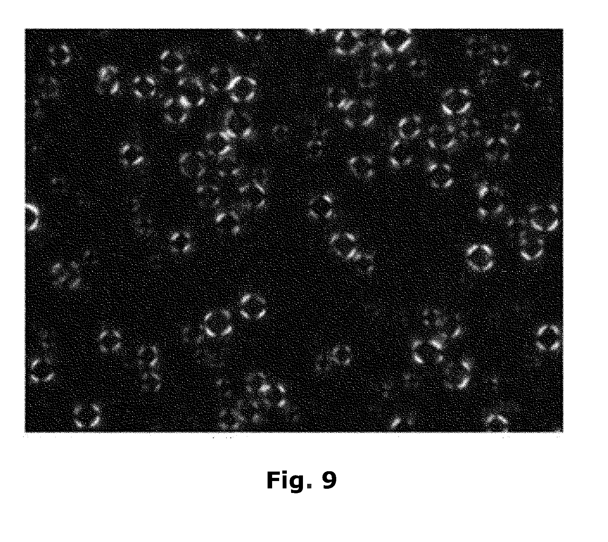

The results of the measurement are shown in Table 3 below. FIGS. 1 to 4 show polarized microscope photographs of the formulations of Examples 5 to 8, respectively, and FIGS. 5 to 9 show polarized microscope photographs of the formulation of Comparative Examples 5, 6, 11 12 and 13, respectively.

As can be seen therein, in the case in which carbomer and the thickener were used in the pre-mix that sufficiently formed the multi-lamellar emulsion, the liquid crystal phase was maintained in the same manner. However, in the case in which lactic acid was used as the organic acid, the liquid crystal phase was slightly poor, and the pre-mixes that did not properly form the liquid crystal phase in the initial stage also showed a poor state. In addition, in the case in which xanthan gum was used as a thickener in the composition of Example 1 without using carbomer or the organic acid, a good liquid crystal phase appeared.

2) pH and viscosity

pH was measured on an undiluted sample using a pH meter (Model 520, Orion), and viscosity was measured using a DV-2 viscometer (Brookfield) under the conditions of spindle #6, 20 rpm and 1 minute.

The results of measurement of pH indicated that all the formulations, excluding Comparative Example 13 in which pH was adjusted, showed a weakly acidic pH of 3-4.

Viscosity was significantly lower in the Comparative Examples than in the Examples.

Table 3: Results of measurement of pH and viscosity

| Samples | pH | Viscosity (cps) | Samples | pH | Viscosity (cps) |
|---|---|---|---|---|---|
| Example 5 | 3.3 | 10,100 | Comparative Example 8 | 3.2 | 5,500 |
| Example 6 | 3.4 | 12,300 | Comparative Example 9 | 3.5 | 9,600 |
| Example 7 | 3.4 | 13,500 | Comparative Example 10 | 3.4 | 10,800 |
| Example 8 | 3.5 | 13,000 | Comparative Example 11 | 3.3 | 4,200 |
| Comparative Example 5 | 3.2 | 3,600 | Comparative Example 12 | 3.4 | 4,400 |
| Comparative Example 6 | 3.3 | 4,900 | Comparative Example 13 | 6.8 | 11,500 |
| Comparative Example 7 | 3.3 | 4,700 | | | |

3) Test for time-dependent stability of formulations

The samples were placed in opaque containers and stored in incubators at 45°C and 4°C and in a temperature cycling chamber (45°C↔5°C; once a day) for 12 weeks, and then the degrees of phase separation and discoloration were evaluated on a four-point scale as follows. The results of the evaluation are shown in Table 4 below.

Criteria for evaluation of formulation separation and discoloration:

0: no change;
1: very slight separation (discoloration);
2: slight separation (discoloration);
3: severe separation (discoloration).

Table 4: Results of time-dependent stability test

| Temperature conditions | Examples 5 to 8 | | | | Comparative Examples 5 to 13 | | | | | | | | |
|---|---|---|---|---|---|---|---|---|---|---|---|---|---|
| | 5 | 6 | 7 | 8 | 5 | 6 | 7 | 8 | 9 | 10 | 11 | 12 | 13 |
| 45°C | 0 | 1 | 0 | 0 | 2 | 2 | 3 | 2 | 2 | 2 | 3 | 2 | 0 |
| 4°C | 0 | 0 | 0 | 0 | 1 | 1 | 1 | 1 | 0 | 0 | 1 | 1 | 0 |
| 45°C↔5°C cycling | 0 | 0 | 0 | 0 | 2 | 2 | 3 | 3 | 3 | 2 | 3 | 2 | 0 |

As can be seen in Table 4 above, in the case of the Examples in which the pH of the pre-mixes that sufficiently formed the multi-lamellar emulsion was adjusted using carbomer and in which a separate thickener was used, the formulations were stable at all 45°C, 4°C and 45°C↔5°C cycling. However, in the compositions in which the formation of the multi-lamellar emulsion itself was incomplete or the pH was adjusted with the organic acid, a separation phenomenon appeared at 45°C and the cycling temperature.

4) Skin irritation evaluation (patch test)

In order to evaluate the skin irritability of the compositions prepared in the Examples and the Comparative Examples, a closed patch test was performed on 10 healthy adult men and women.

Specifically, about 0.5 g of a 1% solution of each sample was added to a chamber, and then attached to the skin using square filter paper having a size of 0.5 cm$^2$ over 4 hours while preventing the loss of the sample, followed by removal of the chamber. At about 1 hour after removal of the chamber, the irritation degree and irritation response of the skin were evaluated by three professional panels according to the criteria shown in Table 5 below, and the skin irritation index was calculated using the following equation. The results of the evaluation are shown in Table 6 below.

Table 5: Criteria for evaluation of skin irritation

| Irritation degree | Irritation response | Criteria for evaluation |
|---|---|---|
| 0 | -- | No symptom |
| 2 | - | Formation of erythema having a size of about 0.1 cm$^2$ |
| 4 | -+ | Formation of erythema and edema having a size of about 0.2 cm$^2$ |
| 6 | + | Formation of erythema and edema having a size of about 0.3 cm$^2$, and formation of blister |
| 8 | ++ | Formation of erythema and edema having a size of about 0.4 cm$^2$, and formation of blister having a size of about 0.3 cm$^2$ |
| 10 | +++ | Formation of large blister having a size of more than 0.3 cm$^2$ |

Equation

Skin irritation index = irritation degree x number of subjects showing irritation response / total number of subjects

Table 6: Results of skin irritation evaluation

| Samples | Evaluation results (number of persons) | | | | | Skin irritation index |
|---|---|---|---|---|---|---|
| | ++ | + | +- | - | -- | |
| Example 5 | 0 | 0 | 0 | 0 | 10 | 0.00 |
| Example 6 | 0 | 0 | 0 | 0 | 10 | 0.00 |
| Example 7 | 0 | 0 | 0 | 0 | 10 | 0.00 |
| Example 8 | 0 | 0 | 0 | 0 | 10 | 0.00 |
| Comparative Example 5 | 0 | 0 | 1 | 2 | 7 | 2.80 |
| Comparative Example 6 | 0 | 1 | 0 | 1 | 8 | 2.80 |
| Comparative Example 7 | 0 | 2 | 0 | 1 | 7 | 3.40 |
| Comparative Example 8 | 0 | 1 | 0 | 2 | 7 | 3.00 |
| Comparative Example 13 | 0 | 0 | 0 | 0 | 10 | 0.00 |

As can be seen from the results in Table 6 above, the Examples of the present invention showed lower skin irritation indices than those of the Comparative Examples. In addition, the sample, to which a separate acidic additive was not added and the pH of which was adjusted to 6.8, also showed no skin irritation.

5) Evaluation of skin moisturizing ability

In order to evaluate the skin moisturizing ability of the samples of the Examples and the Comparative Examples, the content of water in the horny layer of each of 20 adult women was measured using a Corneometer (CM825, Courage-Khazaka Electronic GmbH, Germany). The measurement was performed for 12 hours after application, and the change in the content of water in the horny layer of the skin was calculated using the following equation:

$$\text{Change of skin hydration = skin hydration after certain}$$

$$\text{time - skin hydration before skin barrier damage}$$

The results of the measurement are shown in Table 7 below.

Table 7: Results of measurement of water retention rate

| Measurement results | Examples | | | | Comparative Examples | | | | |
|---|---|---|---|---|---|---|---|---|---|
| | 5 | 6 | 7 | 8 | 5 | 6 | 11 | 12 | 13 |
| Water retention rate | 54 | 48 | 47 | 46 | 38 | 34 | 31 | 34 | 43 |

As can be seen from the results in Table 7 above, the use of carbomer and the multi-lamellar emulsion composition in combination showed a higher water retention rate compared to the use of the organic acid.

6) Evaluation of skin barrier function

In order to evaluate the effects of the samples of Examples 1 to 3 and Comparative Example 13 on a change in the pH of the skin surface and the skin's barrier function, the following experiment was performed.

Each of the samples of Examples 1 to 3 and Comparative Example 13 was applied once to the inside of the upper arm of each of twenty 25-35-year-old women, and then the change in the pH of the skin surface between before and after application was measured. The pH of the skin surface was measured using MPA5 (Courage & Khazaka Electronic GmbH, Germany) equipped with flat type electrodes, and the change in the pH of the skin surface after applying each of the samples of Examples 1 to 3 and Comparative Example 13 twice a day for 1 week was observed. Although the skin to which each of the samples of Examples 1, 2 and 3 was applied showed a slightly low pH, this difference was not significant. The results of the measurement are shown in Table 8 below.

Statistical analysis was performed by two-tailed student's t-test, and a P value < 0.05 was considered statistically significant.

Table 8: Measurement results for pH of skin surface (mean $\pm$ SD) (p values are statistical analysis results with values before application)

| Samples | Before application | After one-time application | After application for 1 week |
|---|---|---|---|
| Example 1 | 5.44 $\pm$ 0.193 | 5.42 $\pm$ 0.182 | 5.35 $\pm$ 0.110 (p=0.078) |
| Example 2 | 5.47 $\pm$ 0.166 | 5.43 $\pm$ 0.125 | 5.39 $\pm$ 0.109 (p=0.080) |
| Example 3 | 5.46 $\pm$ 0.198 | 5.40 $\pm$ 0.159 | 5.37 $\pm$ 0.122 (pH.092) |
| Comparative Example 13 | 5.47 $\pm$ 0.178 | 5.44 $\pm$ 0.139 | 5.49 $\pm$ 0.141 (pH.069) |

The inside of the upper arm of each of the same test subjects was scrubbed with general cleaning soap for 30 seconds twice a day to make bubbles, and then washed with running water for 1 minutes and dried with paper towels.

At 30 minutes after washing, each of the samples of Examples 1 to 3 and Comparative Example 13 was applied to the upper arm. After the same procedure was continued for 1 week, the change in the pH of the skin surface and the change in the skin's barrier function were measured. As a result, it could be seen that the pH of the skin surface in the portion to which the sample of Comparative Example 13 was applied statistically significantly increased, whereas the portion to which the sample of Example 1, 2 or 3 was applied showed no significant increase in the pH of the skin surface compared to before application.

To evaluate the skin barrier function, trans-epidermal water loss (TEWL) was measured using Tewameter TM210 (Courage & Khazaka Electronic GmbH, Germany).

Measurement was performed before application and for 10 days after application, and as a result, all the portions to which Examples 1 to 3 and Comparative Example 13 were applied showed no significant difference between before and after application.

To evaluate the stability of the skin barrier function of the applied portions, the TEWL level was measured while the horny layer in each of the applied portions was repeatedly removed using a D-Squame tape (CuDerm Corporation, Dallas, TX, USA). As a result, it was shown that the increase in the TEWL level in the portion to which each of Examples 1 to 3 was significantly lower than that in the portion to which Comparative Example 13 was applied.

Such results indicate that the samples of Examples 1 to 3 have the effect of increasing the stability of skin's barrier function. The results of the measurement are shown in Tables 9 and 10 below.

Table 9: pH measurement results (mean $\pm$ SD) obtained after application of samples after long-term use of soap

| Samples | Before application | After one-week application |
|---|---|---|
| Example 1 | 5.45 $\pm$ 0.199 | 5.57 $\pm$ 0.198 (p=0.063) |
| Example 2 | 5.45 $\pm$ 0.244 | 5.58 $\pm$ 0.207 (p=0.077) |
| Example 3 | 5.45 $\pm$ 0.226 | 5.59 $\pm$ 0.243 (p=0.067) |
| Comparative Example 13 | 5.45 $\pm$ 0.221 | 5.90 $\pm$ 0.244 (p=4.94E-07) |
| p values are statistical analysis results with values before application | | |

Table 10: TEWL measurement results (mean $\pm$ SD) (g/hm$^2$) for evaluation of stability of skin barrier

| Samples | Before damage | After 5-time application | After 10-time application | After 15-time application |
|---|---|---|---|---|
| Example 1 | 11.49 $\pm$ 1.852 | 15.01 $\pm$ 2.115 | 19.40 $\pm$ 1.933 | 25.17 $\pm$ 2.338 |
| Example 2 | 11.41 $\pm$ 2.074 | 15.11 $\pm$ 1.957 | 19.76 $\pm$ 1.736 | 25.36 $\pm$ 1.816 |
| Example 3 | 11.50 $\pm$ 1.800 | 15.36 $\pm$ 2.311 | 19.89 $\pm$ 1.760 | 25.26 $\pm$ 1.694 |
| Comparative Example 13 | 11.63 $\pm$ 1.630 | 19.46 $\pm$ 2.408 | 25.58 $\pm$ 2.506 | 32.38 $\pm$ 2.451 |

**Industrial Applicability**

As described above, the weakly acidic cosmetic formulation according to the present invention contributes to normalizing the pH of a skin having damaged or reduced skin barrier function, activates lipase that is involved in the synthesis of lipids functioning as a skin barrier, thereby making keratinocytes stable, and inhibits the abnormal proliferation or invasion of skin flora or external bacteria to thereby maintain the skin in a healthy state.

**Claims**

1. A weakly acidic composition for skin external application, comprising: a multi-lamellar emulsion containing a ceramide, a sterol, a fatty acid, a higher alcohol and an emulsifier; a pH adjuster; and a polymeric thickener, the composition having a pH of 3-5.

2. The weakly acidic composition of claim 1, wherein the multi-lamellar emulsion is contained in an amount of 1.0-50.0 wt% based on the total weight of the composition.

3. The weakly acidic composition of claim 2, wherein the multi-lamellar emulsion contains 0.01-5 wt% of the ceramide, 0.1-5 wt% of the sterol, 1-20 wt% of the fatty acid, 1-20 wt% of the higher alcohol, 1-15 wt% of the emulsifier, and a balance of purified water.

4. The weakly acidic composition of claim 2, wherein the ceramide is at least one selected from the group consisting of ceramide-1, ceramide-3, ceramide-3B, ceramide-4, ceramide-6, myristoyl/palmitoyl oxostearamide/arachamide MEA, and dihydroxyisopropyl palmoylpalmamide; the sterol is at least one selected from the group consisting of cholesterol, cholesteryl acetate, cholesteryl sulfate, and phytosterol; the fatty acid or fatty alcohol is a fatty acid or fatty alcohol having 14 to 22 carbon atoms; and the emulsifier is at least one selected from the group consisting of hydrogenated lecithin, polyglyceryl fatty acid ester, sorbitan fatty acid ester, polyoxyethylene glyceryl fatty acid ester, alkyl glucoside, and polyoxyethylene sorbitan fatty acid ester.

5. The weakly acidic composition of claim 1, wherein the pH adjuster is carbomer.

6. The weakly acidic composition of claim 5, wherein the polymeric thickener is at least one selected from the group consisting of hydroxyethyl cellulose, hydroxypropyl cellulose, hydroxypropylmethyl cellulose stearoxy ether, ammonium acryloyldimethyl taurate/VP copolymer, xanthan gum, and acrylate/alkyl acrylate crosspolymer.

7. A cosmetic formulation as a basic cosmetic product or a makeup cosmetic product, which comprises 1-99 wt% of the weakly acidic composition for skin external application according to any one of claims 1 to 6.

**Fig. 1**

**Fig. 2**

**Fig. 3**

**Fig. 4**

Fig. 5

Fig. 6

**Fig. 7**

**Fig. 8**

Fig. 9

## INTERNATIONAL SEARCH REPORT

| | |
|---|---|
| | International application No. |
| | **PCT/KR2014/001289** |

| A. CLASSIFICATION OF SUBJECT MATTER |
|---|
| *A61K 8/18(2006.01)i, A61K 31/164(2006.01)i, A61K 31/20(2006.01)i, A61K 47/38(2006.01)i, A61Q 19/00(2006.01)i* |
| According to International Patent Classification (IPC) or to both national classification and IPC |

| B. FIELDS SEARCHED |
|---|
| Minimum documentation searched (classification system followed by classification symbols) |
| A61K 8/18; A61K 8/49; A61Q 19/00; B01J 13/00; A61K 31/715; A61K 8/14; A61K 8/68; A61K 8/92; A61K 8/60; A61K 8/11; A61K 7/44; A61K 8/19; A61K 31/164; A61K 31/20; A61K 47/38 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: ceramides, sterol, fatty acid, alcohol, multi-lamellar emulsion, pH adjusters, thickener |

| C. DOCUMENTS CONSIDERED TO BE RELEVANT |
|---|

| Category* | Citation of document, with indication, where appropriate, of the relevant passages | Relevant to claim No. |
|---|---|---|
| Y | KR 10-2002-0070154 A (NEOPHARM CO., LTD.) 05 September 2002<br>See abstract; claims 1-3, 8 and 11; line 25 on page 13-3 - line 7 on page 13-5; table 1. | 1-7 |
| Y | KR 10-2010-0114416 A (KOREA KOLMAR HOLDINGS CO., LTD) 25 October 2010<br>See abstract; claims 1-9; paragraph [0013]. | 1-7 |
| Y | KR 10-2012-0103768 A (AMOREPACIFIC CORPORATION) 20 September 2012<br>See abstract; claims 1 and 3. | 5-6 |
| A | KR 10-2009-0056293 A (NEOPHARM CO., LTD.) 03 June 2009<br>See the entire document. | 1-7 |
| A | KR 10-2007-0117800 A (NEOPHARM CO., LTD.) 13 December 2007<br>See the entire document. | 1-7 |
| A | US 5985177 A (YOSHIDA, Katsunori et al.) 16 November 1999<br>See the entire document. | 1-7 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 18 JUNE 2014 (18.06.2014) | **19 JUNE 2014 (19.06.2014)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br>Telephone No. |
|---|---|

Form PCT/ISA/210 (second sheet) (July 2009)

INTERNATIONAL SEARCH REPORT
Information on patent family members

International application No.

**PCT/KR2014/001289**

| Patent document cited in search report | Publication date | Patent family member | Publication date |
|---|---|---|---|
| KR 10-2002-0070154 A | 05/09/2002 | CN 1198588 C | 27/04/2005 |
| | | CN 1372922 A | 09/10/2002 |
| | | EP 1236462 A1 | 04/09/2002 |
| | | EP 1370241 A1 | 17/12/2003 |
| | | EP 1370241 A4 | 26/01/2005 |
| | | JP 03887182 B2 | 28/02/2007 |
| | | JP 04343533 B2 | 14/10/2009 |
| | | JP 2002-255792 A | 11/09/2002 |
| | | JP 2004-520408 A | 08/07/2004 |
| | | TW 1248369 B | 01/02/2006 |
| | | WO 02-067900 A1 | 06/09/2002 |
| KR 10-2010-0114416 A | 25/10/2010 | KR 10-1094477 B1 | 19/12/2011 |
| KR 10-2012-0103768 A | 20/09/2012 | NONE | |
| KR 10-2009-0056293 A | 03/06/2009 | NONE | |
| KR 10-2007-0117800 A | 13/12/2007 | KR 10-0840905 B1 | 24/06/2008 |
| | | US 2007-0286835 A1 | 13/12/2007 |
| US 05985177 A | 16/11/1999 | EP 0782846 A2 | 09/07/1997 |
| | | EP 0782846 A3 | 14/01/1998 |
| | | EP 0782846 B1 | 09/10/2002 |
| | | JP 03306286 B2 | 24/07/2002 |
| | | JP 03340310 B2 | 05/11/2002 |
| | | JP 08-323188 A | 10/12/1996 |
| | | JP 09-255562 A | 30/09/1997 |
| | | JP 09-276676 A | 28/10/1997 |
| | | JP 10-028858 A | 03/02/1998 |
| | | KR 10-0509852 B1 | 06/07/2006 |
| | | TW 470653 A | 01/01/2002 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- The Characterization of Molecular Organization of multilamellar Emulsions Containing Pseudoceramide and Type III Synthetic Ceramide. *J Invest Dermatol.,* October 2003, vol. 121 (4), 794-801
- Effects of soaps and detergents on skin surface pH, stratum corneum hydration and fat content in infants. *Dermatology,* 1997, vol. 195, 258-262
- The effect of detergents on skin pH and its consequences. *Clin Dermatol.,* 1996, vol. 14 (1), 23-27

- **PETER M. ELIAS.** *Abnormal skin barrier in the pathogenesis of atopic dermatitis*
- Stratum Corneum pH in Atopic Dermatitis. *Am J Clin Dermatol,* 2004, vol. 5 (4), 217-223
- **ANNA DI NARDO ; RICHARD L. GALLO.** *Skin barrier: Cutaneous barriers in defense against microbial invasion,* 364-365